# EUROPEAN PATENT SPECIFICATION

(11) **EP 0 548 070 B1**
(45) Date of publication and mention of the grant of the patent: **06.08.1997**
(21) Application number: 90907691.1
(22) Date of filing: 20.04.1990
(51) Int. Cl.: C12P 21/08, A61K 39/395, G01N 33/574, G01N 33/577, G01N 33/68

(54) **MONOCLONAL ANTIBODY FOR DIFFERENTIATION OF SQUAMOUS CELL CARCINOMA ANTIGENS AND METHOD OF USE FOR SAME**
MONOKLONALER ANTIKÖRPER ZUR DIFFERENZIERUNG VON "SQUAMOUS CELL CARCINOMA"-ANTIGENEN UND VERFAHREN ZU DESSEN VERWENDUNG
ANTICORPS MONOCLONAL POUR LA DIFFERENCIATION D'ANTIGENES DE CARCINOMES DE CELLULES SQUAMEUSES ET PROCEDE D'UTILISATION

(30) Priority: 21.04.1989 US 341402
(43) Date of publication of application: 30.06.1993
(73) Proprietor: BIOMIRA, INC., Edmonton Alberta T6N 1E5 (CA)
(72) Inventor: Samuel, John, Edmonton, Alberta T6H 5B5 (CA); Longenecker, Bryan Michael, Edmonton, Alberta T6G 1T3 (CA); Stanczyk-Brzezinska, Grazyna, Edmonton, Alberta T6H 5A1 (CA); Willans, David, Edmonton, Alberta T6H 5X4 (CA); Honore, Louis H., Edmonton, Alberta T6J ON2 (CA); Haines, Deborah M., Saskatoon, Saska S7N 0J9 (CA); Diener, Erwin, Edmonton, Alberta T6G 1T7 (CA); Ding, Lei, Edmonton, Alberta T6H 3C8 (CA)
(74) Representative: Andersen, Henrik Rastrup
(86) International application number: US9002152
(87) International publication number: WO9012885

(56) References cited:
- DIFFERENTIATION, vol. 31, 1986, Springer-Verlag, Berlin (DE); M. HUSZAR et al. , pp. 141-153 #
- CANCER RESEARCH, vol. 47, no. 12, 15 June 1987, Philadephia, PA (US); D.A. JOHNSON etal., pp. 3118-3122
- JOHNSON et al., pp. 3118-3122
- CANCER RESEARCH, vol., 49, no. 9, 01 May 1989, Philadelphia, PA, US); J. SAMUEL et al., pp. 2465-2470
- BIOSIS, AN no. 88.477505, J. Samuel et al. : "Novel squamous cell carcimona differentiation antigens recognized by MAb 174H.64", see abstract T115

## Description

### BACKGROUND OF THE INVENTION

### 1. Field of the Invention

This invention relates to a specfic binding antibody for differentiation of squamous cell carcinoma antigen and a method for using the monoclonal antibody. Specifically, this invention relates to a murine monoclonal antibody for differentiation of a squamous cell carcinoma antigen of cytoskeletal proteins having molecular weights of 48 to 50 KD and 57 KD and a method for using the monoclonal antibody.

### 2. Description of the Background Art

G. Kohler and C. Milstein reported a lymphocycte fusion method in their article, "Continuous Cultures of Fused Cells Secreting Antibodies of Preferred Specificity", Nature 265:495 (1975). Their work produced monoclonal antibodies by fusing mouse myeloma tumor cells to spleen cells derived from a mouse that was previously immunized with an antigen. This discovery of antibody production provides a method for unlimited supply of pure antibodies and antibody reagents. These antibodies provide useful tools for cancer diagnosis and therapy.

A multilayered epithelia is found in the epidermis, the mouth and oral cavity, the esophagus, the epiglottis, the larynx, the pharynx, the vagina, and the cervix. These are areas where squamous cell carcinoma and precursor cells of the transformation process are located. Precursor cells are squamous cells, in any stage of transformation, that are committed to become invasive squamous cell carcinomas. This "pre-SCC stage" is a stage occurring prior to cells being in "frank malignancy" or cancerous cells. The pre-SCC stage includes squamous cells which are morphologically normal, atypia (hyperplasia, metaplasia, hyperkeratosis), significant atypia, dysplasia (mild dysplasia - CIN 1, moderate dysplasia-CIN II, and severe dysplasia - CIN III) or carcinoma in situ (CIS). Benign squamous cells in the pre-SCC stage are not committed to become invasive squamous cell carcinomas.

Squamous cell carcinoma (SCC) is a common histological type of cancer of the head and neck, lung, oral cavity, or cervix. Identification and characterization of molecular markers associated with squamous cell carcinoma facilitates earlier diagnosis and treatment of squamous cell carcinomas. Functional studies on squamous cell carcinoma markers can aid in better understanding of the biology of this type of cancer.

Monoclonal antibodies (MAbs) are useful tools as molecular and functional probes for studying tumor associated antigens. The majority of the monoclonal antibodies raised against squamous cell carcinomas are produced by using "squamous cell carcinoma cell lines as the immunogen. Antigenic molecules defined by such monoclonal antibodies include cell-surface proteins and cytoskeletal components. Several monoclonal antibodies reacting with squamous cell carcinoma have been reported. However, many of these antibodies either show cross reactivity with other types of cancers or their reactivity is limited to squamous cell carcinomas of certain organs only.

Antibodies have been developed against keratinocytes and their reactivity with squamous cell carcinomas evaluated. A monoclonal antibody produced against human keratinocytes, VM-2, has been reported to selectively bind to the basal layer of the epidermis, as well as squamous and basal cell carcinomas. This antibody is disclosed in two articles: Morhenn, et al., "A Monoclonal Antibody Against Basal Cells of Human Epidermis: Potential Use in the Diagnosis of Cervical Neoplasis" J. Clin. Invest., 76: 1978-1983, 1985 and Oseroff, et al., "Use of Murine Monoclonal Antibody Which Binds to Malignant Keratinocytes to Detect Tumor Cells in Microscopically Controlled Surgery" J. Am. Acad. Dermatol.. 8: 616-619, 1983.

In Differention, **31** (1986) p. 141-153 monoclonal antibodies K_{S} 8.12 and K_{K} 8.60 are disclosed which bind to the suprabasal layer of skin and vaginal epithelium.

The Mab PFI/D as disclosed in Cancer Research, **47** (1987) p. 3118-3122 is reactive with non-squamous lung adenocarcinomas and small cell lung carcinomas.

Monoclonal antibodies developed against cytokeratins have been used as probes for studying the keratin expressions in normal and neoplastic epithelia. Cytokeratins exhibit a differentiation-specific pattern of distribution in different layers of stratified epithelium. Specific keratin polypeptides have also been reported as molecular markers for squamous cell carcinoma. Antikeratin MAbs have been proposed as tools for diagnosis of tumors including squamous cell carcinomas.

Antibodies that are known to react with squamous cell carcinoma antigens include MAbs AE1 and AE3, MAb 17.13, and MAbs, PKK1 and PKK2. These antibodies have been described by numerous sources including those identified below. These antibodies fail to differentiate the cytoskeletal proteins with a molecular weight of approximately 48 to 50 kD and 57 KD.

The MAbs AE1 and AE3 are described by: T.T. Sun, et al., J. Invest. Dermatol., 81: 1095 (1983); W. G. Nelson, et al., Cancer Res. 44: 1600 (1984); Hybritech, Inc. (Hybritech), product insert provided with MAb AE1 and AE3; and J. Reibel, et al., Acta Path. Microbiol. Immunol. Scand. Sect. A 93: 323 (1985). The tissue specificity for MAb AE1 is the epidermal basal layer and almost all epithelia. The tissue specificity for MAb AE3 is entire epidermis and all epithelia. MAb AE1 binds squamous cell carcinoma as well as non-SCCs (e.g., adenocarcinoma). No information is available on staining of a squamous cell carcinoma using MAb AE3 alone. MAb AE1 recognizes keratins of acidic type (Mr 40, 50 and 56.5 KD), whereas MAb AE3 recognizes keratins of basic type (58, 52 and 65-67 KD). Hybritech claims "a combination of these two antibodies recognizes almost all known human epithelia keratins." Since these antibodies have a broad spectrum of reactivity towards keratins, they are useful for distinguishing epithelia tissues and their neoplasms from non-epithelial tissues and their neoplasms. However, neither of these antibodies can distinguish "stratified squamous epithelia" and neoplasms derived from them from other types of epithelia (e.g., simple epithelia) and tumors of other epithelia origin.

The MAb 17.13 is described by R. Rankin, et al., Cancer Res. 47: 5684 (1987) and published European Patent Application (InTek Diagnostic) Number 0 275 705 (The latter reference summarizes the characteristics of many anti-SCC antibodies.) No characterization of the antigen recognized by this antibody has been reported. The antigen is presumed to be an intermediate type filament, based on electron microscope data. The European patent application states that the antigen is not a cytokeratin. The antibody is specific for basal layer of stratified squamous epithelia. Its reactivity with precancerous lesions of CIN (Stages I-III) is approximately 30% positive. Molecular weight and other characteristics of the MAb 17.13 antigen have not been reported. The SCC-reactivity of MAb 17.13 is homogeneous. The reactivity of MAb 17.13 with animal tissues is unknown.

The MABs PKK1 and PKK2 are described by Holfhofer, et al., Lab. Invest. 49: 317 (1983) and product inserts of LabSystems, Inc. (Chicago, Illinois) for MAbs PKK1 and PKK2. These antibodies, respectively, recognize the PKK1 antigen which is a cytokeratin (Mr 41 KD, 45 KD, 48 kD and 56 kD) and the PKK2 antigen for which information is not available. MAb PKK1 binds to all layers of stratified epithelia. PKK2 reportedly (according to Intek) binds to basal and parabasal layers of stratified squamous epithelia as well as columnar epithelium of the normal lung. MAb PKK2 binds to SCCs, but also binds to bronchioalveolar carcinoma. MAb PKK1 binds to all layers of stratified epithelia and reacts with a broad range of cytokeratins. MAb PKK2 also shows broad reactivity in that it is reactive with normal columnar epithelium (lung) as well as stratified epithelium. Thus, it fails to distinguish stratified squamous epithelia from other types of epithelia. Besides, its tumor reactivity also lacks specificity in that it reacts with lung tumors of nonSCC type.

The art lacks an antibody preparation that is specific for squamous cell carcinoma antigens in many organs and which is useful as a therapeutic and diagnostic agent.

### SUMMARY OF THE INVENTION

The invention is an antibody preparation which essentially only recognize antigens associated with squamous cell carcinoma (SCC) and stem cell populations of stratified epithelium. Preferably, this preparation comprises monoclonal antibody MAb 174H.64 or a specific binding fragment thereof. The antigens recognized by MAb 174H.64 include cytoskeletal proteins with molecular weights of approximately 48 to 50kD and 57kD. Mab 174H.64 was disclosed in BIOSIS, AN No. 88.477 505 (Abstract T 115) as being capable of binding to SCC antigens having molecular weights of 125, 135 and 245 KD, respectively.

The invention includes methods for using the antibody for diagnosis of squamous cell carcinoma tumors by immunohistology and immunodetection of circulating SCC antigens in sera. The invention further includes use of the antibody in the manufacture of compositions for non-invasive diagnostic immunoimaging in vivo as well as treatment of squamous cell carcinoma tumors using antibodies conjugated to cytotoxic drugs or tagged with a radiosotope, and as a research tool for studying the normal and pathological conditions of stratified squamous epithelium.

### BRIEF DESCRIPTION OF THE DRAWINGS

Figure 1 illustrates the pattern of MAb 174H.64 binding to squamous cell carcinoma tissues. The peripheral layer of a -human anorectal SCC is selectively stained by immunoperoxidase.

Figure 2 compares the specificity of cell killing of the different antibodies and antibody conjugates with DM alone.

Figure 3 illustrates uptake of ¹²⁵I-IUdR relative to SCC cells injected.

Figure 4 shows the reduction in uptake as a result of various pretreatments of the SCC cells.

Figure 5 shows the uptake when, after injection of SCC cells into mice, the mice are treated with DM, MAb 174H.64, or a MAb 174H.64-DM conjugate.

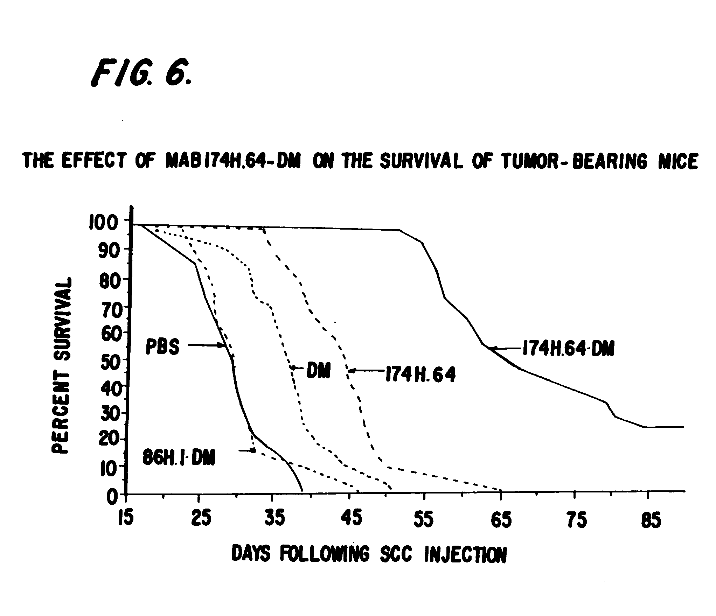

Figure 6 illustrates the effect of MAb 174H.64-DM on survival of tumor bearing mice.

Figure 7 is a photograph of a sdan of a patient's lung, imaging a metastasis of a cervical carcinoma by means of a MAb 174H.64-DM conjugate.

### DETAILED DESCRIPTION OF THE INVENTION

The invention includes monoclonal antibody preparations. The term "antibody preparations" encompasses whole antibodies according to this invention as well as specific binding fragments of such antibodies. An antibody or antibody fragment according to this invention can be conjugated to another substance. Such substances include labels (including, without limitation, radioisotopes, enzymes, co-enzymes, enzyme inhibitors, fluorophores and fluorescence quenchers), cytotoxic drugs such as daunomycin, and macromolecular chromatographic supports.

The invention includes a murine monoclonal antibody for differentiation of squamous cell carcinoma antigens and a method for using the monoclonal antibody. The monoclonal antibody of this invention selectively or, essentially, only recognizes antigens associated with squamous cell carcinoma and stem cell populations of stratified epithelium. The antibody, in well-differentiated squamous cell carcinoma tumors, preferentially binds with the peripheral proliferative compartment of the tumor. The antigens recognized by the monoclonal antibody of this invention include cytoskeletal proteins with molecular weights of approximately 48 to 50kD and 57kD. The potential applications of the antibody include diagnosis of squamous cell carcinoma tumors by immunohistology, noninvasive diagnostic imaging in vivo, treatment of squamous cell carcinoma tumors using monoclonal antibbdies conjugates of radionuclide or cytotoxic drugs, and as a research tool in studying the normal and pathological conditions of -stratified squamous epithelium. The monoclonal antibody, though raised against Tn-HSA, does not demonstrate any reactivity with this non-SCC immunogen and demonstrates a high degree of selectivity towards squamous cell carcinoma tissues.

The preferred monoclonal antibody of this invention is designated MAb 174H.64. The description of this invention shall refer primarily to this preferred monoclonal antibody. The reference to the preferred embodiment of the invention in this description is not intended to exclude essentially equivalent variations of the preferred monoclonal antibody from the invention. The organ of origin can include the mouth and oral cavity, the esophagus, the epiglottis, the larynx, the pharynx, the vagina, and the cervix.

MAb 174H.64 selectively binds with human squamous cell carcinoma (100% in the inventors' studies) irrespective of the organ of origin. This antibody does not bind with tumors derived from simple epithelia or tumors of non-epithelial origin. This squamous cell carcinoma-specific reactivity of the antibody extends to spontaneous SCC-like tumors of other mammals including cows, dogs and cats. No data is available for the reactivity of MAb 17.13 and VM-2 with animal tumors.

MAb 174H.64 selectively binds with a stem cell population (basal and parabasal layers) of normal human stratified squamous epithelium. The antibody also shows selective reactivity with thymic epithelium and myoepithelial cells of breast ducts. The antibody does not bind with simple epithelia or non-epithelial tissues. This suggests that MAb 174H.64 recognizes a differentiation antigen characteristic of stem cell populations of stratified squamous epithelia. MAb 17.13 and VM-2 also show selective reactivity with the basal layer of stratified Squamous epithelium. MAb 17.13 has also been reported to be reactive with myoepithelial cells of human breast ducts. No data is available for the reactivity of these two antibodies with thymic epithelium and for the reactivity of VM-2 with myoepithelial cells.

Squamous cell carcinoma tumors with high degree of differentiation show preferential binding of the antibody towards the peripheral layer of the tumor comprised of highly proliferative primitive cells. The more differentiated cells, towards the center of the tumor show little or no staining with the antibody. This observation suggests that the epitope recognized by MAb 174H.64 is characteristic of the proliferative compartment of the squamous cell carcinoma and is not expressed by the more differentiated squamous cell carcinoma tumor cells. This is surprising in that it is believed that no other monoclonal antibody has such selective reactivity towards the proliferative compartment of squamous cell carcinoma. This characteristic can provide a valuable diagnostic and therapeutic utility for the invention.

MAb 174H.64 shows a unique pattern of staining with normal human cervix. The reactivity of MAb 174H.64 with basal and parabasal layers of mature squamous epithelium is 97 and 81%, respectively, whereas the reactivity is reduced to about 73 and 59%, respectively, in immature squamous epithelia. The subcolumnar (reserve) cells of the cervix show a reactivity of 70%. These results suggest that the differentiation antigen recognized by MAb 174H.64 is also a marker for epithelia maturation in the cervix.

MAb 174H.64 shows binding with premalignant lesions. In cervical intraepithelial neoplasia (CIN) 69% of the cases showed reactivity with basal layer and 54% of the cases showed reactivity with parabasal layer. In kilocytotic atypia (KA) all of the cases tested showed reactivity with the antibody.

The antigens recognized by MAb 174H.64 are two cytoskeletal proteins with approximate molecular weights of 48 to 50kD and 57kD. These molecular weights, although suggestive of human cytokeratins #14 and #5 which are markers for non-keratinizing stratified squamous epithelium, are different since the cytokeratins #14 and #5 are distributed also in suprabasal layers.

The monoclonal antibody of this invention can be useful as a diagnostic reagent, a therapeutic agent for squamous cell carcinoma tissue, and a research tool. The highly selective reactivity of MAb 174H.64 can be used as a reagent for unambiguous diagnosis of squamous cell carcinomas irrespective of the tissue of origin. This diagnostic utility can include immunohistological diagnosis, diagnostic imaging, and serodiagnosis. The therapeutic utility can include specific use as a target cytotoxic drug. The research utility can include numerous uses regarding cell studies and therapeutics. The antibody may also be used in the immunopurification of SCC-associated antigens. The purified antigens may be used to raise monospecific monoclonal antibodies for use as taught herein.

MAb 174H.64 can be used as a reagent for histological typing of tumors in frozen sections. For example, the diagnosis of adenosquamous carcinoma of cervix can be difficult with respect to ascertaining whether undifferentiated areas are of squamous or glandular derivation. A positive staining with MAb 174H.64 can resolve this problem, which may be difficult even with electron microscopy. For such diagnostic purposes, any labeling technique suitable for histological use may be employed, including but not limited to radiolabels, fluorophores, enzymes, and ferritin. The labeling may be direct or indirect (e.g., via an avidin-biotin, protein A-F_{c}, or antibody -F_{c} bridge).

MAb 174H.64 can be used for diagnostic imaging of squamous cell carcinoma. Unambiguous diagnosis of squamous tumors based on selective in vivo localizations of radiolabelled MAb 174H.64 can be especially valuable for the following applications. This procedure can determine that location, size, and extent of the invasion of the primary tumor. It can track the metastatic spread of the tumor and detect small metastatic areas in lymph nodes or organs, thus preventing the need for exploratory surgery. The procedure provides non-invasive determination of the histogenesis of a tumor. It can also provide a noninvasive monitoring of the response of the tumor to treatment. The localization of the monoclonal antibody in a tumor, that is necessary for use in diagnostic imaging, is demonstrated in Table 1. The acutal imaging of a tumor is set forth in Example 3.

**TABLE 1**

| Biodistribution of ¹²⁵I - 174H64 and ¹³¹I - MOPC-21 (isotype matched control antibody) in normal and tumor bearing (KLN-205 a murine squamous cell carcinoma) lung of DBA2 mice 72h after the injection of the antibodies. | | | | |
|---|---|---|---|---|
| | % Injected dose/g tissue | | % Injected dose in the organ | |
| | 174H.64 | MOPC-21 | 174H.64 | MOPC |
| Tumor Bearing Lung | 7.7 | 1.6 | 5.4 | 1.16 |
| | ±0.99 | ±0.51 | ±2.85 | ±0.97 |
| Normal Lung | 5.5 | 1.9 | 0.94 | 0.34 |
| | ±0.209 | ±1.3 | ±0.062 | ±0.24 |

Serodiagnosis can be performed, if the antigens detected by MAb 174H.64 are shed into serum. Detection is then possible by the ability of the serum to bind the monoclonal antibodies using ELISA, RIA or other immunoassay techniques. This principle can also be extended, as a diagnostic tool, to detection of the antigen shed into urine in bladder squamous cell carcinomas, or of antigen shed in other biological fluids as appropriate.

The monoclonal antibody of this invention can provide specific therapy for squamous cell carcinoma. The monoclonal antibody in such therapy can be used alone, or to deliver a cytotoxic or radiolabelled conjugate to the target SCC cells. Studies with a daunomycin conjugate of this antibody demonstrate the conjugate's ability to kill a murine squamous cell carcinoma cell line in a selective fashion. Tables 2 and 3 demonstrate this therapeutic utility.

**TABLE 2**

| Hematopoietic reconstitution of lethally irradiated DBA2J mice with syngeneic bone marrow from which contaminating KLN-205 squamous cell carcinoma cells had been purged with a target-specific immunoconjugate (174H.64 - Daunomycin) | |
|---|---|
| Treatment Groups | cpm in Lung means ± SD |
| bone marrow only | 1,372 ± 449 |
| bone marrow + KLN-205 (untreated) | 29,336 ± 4,505 |
| bone marrow + KLN-205 (DM treated) | 11,065 ± 6,144 |
| bone marrow - KLN-205 (antibody treated) | 29,197 ± 2,490 |
| bone marrow + KLN-205 (immunoconjugate treated) | 4,207 ± 2,121 |
| A mixture of 1 x 10⁶ DBA2J bone marrow cells and 1 x 10⁶ KLN-205 cells was treated in vitro for 2 hours at 4°C washed and injected into each of 850 rad irradiated recipients. Twenty-five days later, lungs were tested for ¹²⁵I-IUdR incorporation by injecting 2 µCi of ¹²⁵I-IUdR and counting the lungs for ¹²⁵14 h after the injection. The concentration of the immunoconjugate was adjusted to contain 10µg/ml of DM. | |

**TABLE 3**

| Effect of MAb 174H.64 Daunomycin (DM) conjugate in vivo treatment, as the KLN-205 SCC tumor growth in DRA27 mice. | | |
|---|---|---|
| Group | Number of Animals | CPM (¹²⁵I-IUdR Uptake) |
| Normal | 4 | 8.038 ± 3,471 |
| 174H.64 Daunomycin Conjugate | 5 | 32,668 ± 20,354 |
| Untreated | 3* | 108,464 ± 82,570 |
| * Two of the untreated mice died on day 30 of the experiment and had extensive tumor spread in the lung. | | |
| * KLN-205 (10⁶ cells) were injected into DBA2 male mice and were divided into two groups (5 mice each) Five of the above mice each were injected with MAb 174H.64-DM (100µg DM, 1.8 mg of MAb, on the 5th and the 10th days after the injection of KLN-205 cells. On the 34th day 2 µCi of ¹²⁵I-IUdr was injected intravenously to each mice and lungs were taken out and counted in a gamma counter. | | |

The present invention is not limited to the use of a particular cytotoxic drug, though daunomycin is preferred. Other cytotoxic drugs known to be effective against cancer cells include ricin. Nor is it limited to any particular method of conjugating the cytotoxic drug to the antibody, though use of an acid-sensitive cis-aconityl spacer is preferred.

MAb 174H.64 can be labelled with radionuclides such as ¹³¹I, ¹²⁵I, ⁹⁰Y, ²¹¹At, ⁶⁷Cu, and ¹⁸⁶Re and used to deliver therapeutic doses of radiation selectively to the squamous cell carcinoma tumor tissue. For the purpose of the appended claims, reference to the antibody as being associated with a cytotoxic agent is intended to include antibodies labeled with a therapeutic radionuclide. MAb 174H.64 can be used for imaging when labelled with radionuclides such as ⁹⁹Tc, ¹¹¹In, and ⁶⁷Ga. The present invention is not limited to use of a particular radionuclide or radiolabeling technique.

The monoclonal antibody of this invention can be used as a research tool as follows. Expression of the cytoskeletal proteins can be detected by MAb 174H.64 during differentiation of the normal stratified epithelium and used to establish its functional significance. Evaluation can be performed of premalignant lesions of uterine cervix such as CIN and KA. This monoclonal antibodies binds with 69% of basal CIN and all of KA tissue samples tested. Clinical and therapeutic significances of positivity and negativity can be determined by careful clinical and pathological follow up. The prognosis of the lesion can be defined in terms of negativity or grades of positivity to this antibody. The monoclonal antibody can be used to study the biology of squamous cell carcinoma with respect to expression of the antigen during transformation, progression, and differentiation of the tumor.

The monoclonal antibody of this invention can be made and used according to the following example. A hybridoma cell line secreting MAb 174H.64 was deposited under the Budapest Treaty on April 18, 1989 at the American Type Culture Collection (ATCC), Rockville, Maryland, and has received the designation ATCC HB 10105.

### Example 1: Preparation and Characterization of MAb 174H.64

The example is of the preferred embodiment of the invention. The preferred embodiment of the invention is the monoclonal antibody designated MAb 174H.64. The following procedure represents the best mode for deriving and testing the monoclonal antibody of this invention. The comparative data are provided for comparison purposes to demonstrate certain unexpected and desirable characteristics of the invention.

### Immunization and hybridoma production

RBF/Dn mice were immunized by two consecutive i.p. injections, one week apart, of a mixture of human Choriogonadotrophin (HCG, 50 µg) and a synthetic carbohydrate conjugated to human serum albumin. This conjugate (Tn-HSA) is N-acetyl galactosamine alpha-linked to human serum albumin at a ratio of 40 residues per molecule of carrier (50 µg) in complete Freund's adjuvant. On the 12th day they were given another i.p. injection of a mixture of HCG (400 µg) and Tn-HSA (400 µg) in phosphate-buffered saline (PBS, contains 140 mM NaCl, 2.68mM KCl, 8.1 mM Na₂HPO₄ . 2H₂O, 1.4 mM KH₂PO₄, pH 7.2). On 13th day they were further immunized by an i.v. and an i.p. injection, each consisting of a mixture of HCG and Tn-HSA (200 µg each). The latter procedure was repeated on the 14th day. On the 15th day, the spleen cells from mice having high antisera titres against HCG and Tn-HSA were fused with FOX-NY cells (Hyclone Lab., NY). Hybride with positive reactivity against Tn-HSA and negative reactivity with HSA were selected and recloned. The supernatants from the reclones were tested for reactivity against tumor tissues by immunohistological staining. A reclone designated 174H.64 showed strong and selective reactivity with human squamous cell carcinomas, although it did not show any reactivity with Tn-HSA. This hybridoma (double recloned) was chosen for further studies based on its selective reactivity with squamous cell carcinoma tissues.

### Isotyping

MAb 174H.64 was isotyped using a Mouse Type Isotyping Kit (Biorad Laboratories, Mississauga, Ontario, Canada) by an enzyme immunoassay (EIA) method. A microtitre plate was coated with the antibody by an overnight incubation of 4°C of the culture supernatant of the hybridoma grown in serum free medium. The microtitre wells were serially incubated with bovine serum albumin (1%) in PBS for 30 minutes at 37°C, isotyping reagent (rabbit anti mouse Ig subtypes) for two hours at room temperature, and goat anti rabbit IgG conjugated with horseradish peroxidase (HRP) for 1 hour at room temperature. After each of the above steps, the plate was washed with PBS. Finally, the microtitre wells were incubated with HRP substrate solution for 30 minutes at room temperature and the optical density read by an EIA reader.

### Purification of 174H.64.R24; Radiolabelling and Biotinylation

Ascitic fluids were produced by injection of hybridoma 174H.64 recloned cells reclone 174H.64.R24 (2 x 10⁵) i.p. into pristane-primed Balb/c mice. The antibody was purified by a two step procedure involving ammonium sulphate (50%) precipitation and protein A sepharose column chromatography. The purified antibody fraction contained a single protein band on cellulose acetate electrophoresis and was immunoreactive on squamous cell carcinoma frozen sections as shown by immunoperoxidase staining.

The antibody was radioiodinated by the iodogen method. Purified MAb 174H.64.R24 (50 µg) in phosphate buffered saline (PBS, 50 µg) was mixed with "no carrier added" Na ¹²⁵I (500 Ci) in PBS (10 µl) and was transferred into a borosilicate glass tube coated with iodogen (2 µg) at the bottom. The reaction was allowed to proceed at room temperature for 2 minutes and the solution was then transferred into another tube containing 0.05% KI in PBS (100/µl) and allowed to equilibrate for 3 minutes the antibody was separated from inorganic iodide using a Biogel™ P-100 spun column.

MAb 174H.64 was biotinylated according to a known, reported procedure. A solution of MAb 174H.64 (2 mg) in PBS (400 µl) was mixed with 0.2 M sodium bicarbonate solution containing. 0.15 M potassium chloride pH 8.1. To this a solution of Biotin-LC-NHS (125 µg, Pierce) in PBS (25 µl) was added and incubated at room temperature for 20 minutes. The reaction was stopped by addition of 1 M ammonium chloride solution pH 6.0 (50 µg) and the solution was extensively dialyzed against PBS.

### Cell Lines

All the squamous cell carcinoma cell lines were obtained from American Type Culture Collection, Rockville, Maryland, and grown in RPMI containing 10% fetal calf serum (FCS) and gentamicin (40 units/ml). Normal human keratinocytes were obtained from Clonetics Corporation, San Diego, CA, and were cultured in serum free keratinocyte growth medium (Clonetics Corporation).

### Isolation of mouse epidermal keratinocytes

Epidermal keratinocytes were isolated from neonatal (1 day old) ICR mice according to a known, reported procedure. Briefly, mice were decapitated, skin was removed and washed in Hanks balanced salt solution (HBSS). The skin was then placed dermal side down in HBSS containing trypsin (0.025%) EDTA (0.02%) and incubated at 37°C for 1 hour. The trypsin EDTA solution was aspirated and replaced with RPMI with 10% FCS. The epidermis was then gently separated from the dermis using forceps and agitated gently in the medium. The resulting cell suspension was then separated from the epidermal and dermal layers and washed 2 times with RPMI followed by PBS. This cell suspension was used for immunoperoxidase staining.

### Immunohistology

Tumor tissues for immunohistological studies were embedded in OCT, frozen in isopentane and stored at 70°C. Immunoperoxidase staining of frozen sections or cell smears were done according to the avidin-biotin peroxidase complex (ABC) method that is known in the art. Frozen sections of 8 um were cut on a cryostat and mounted on poly L-lysine (PLL) coated glass slides. The cell smears were prepared from a single cell suspension of cells using PLL coated glass plates and were air dried. The glass slides were then serially incubated with primary antibody (174H.64) for 15 minutes, with 0.02% glutaraldehyde for 5 minutes at 4°C, with biotinylated secondary antibody (antimouse IgG) for 30 minutes, and with ABC reagent for 20 minutes. After each of the above steps, the slides solution containing 0.5% hydrogen peroxide for 5 minutes, washed in water, counterstained with hematoxylin and mounted in permount. A brown color in the cells corresponds to positive reaction with the antibody. Whenever mouse tissues were stained by the above procedure, a biotinylated primary antibody was used and the step with the antimouse IgG was omitted. An isotype matched mouse myeloma protein (MOPC-21) was used as a negative control antibody in all immunoperoxidase staining.

### Reactivity of MAb 174H.64 With Cancer Tissues

Frozen sections of human cancer tissues of various origins were tested for reactivity with MAb 174H.64 by immunoperoxidase staining by the ABC method. An isotype matched control antibody (MOPC-21) was used as a negative control. The results of these studies are summarized in-Table 4.

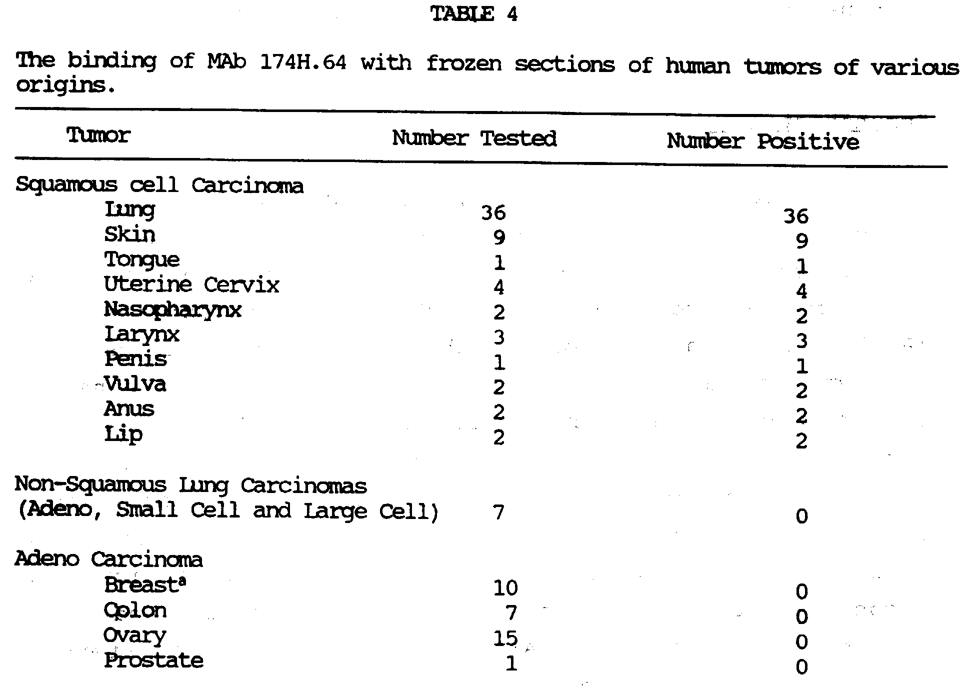

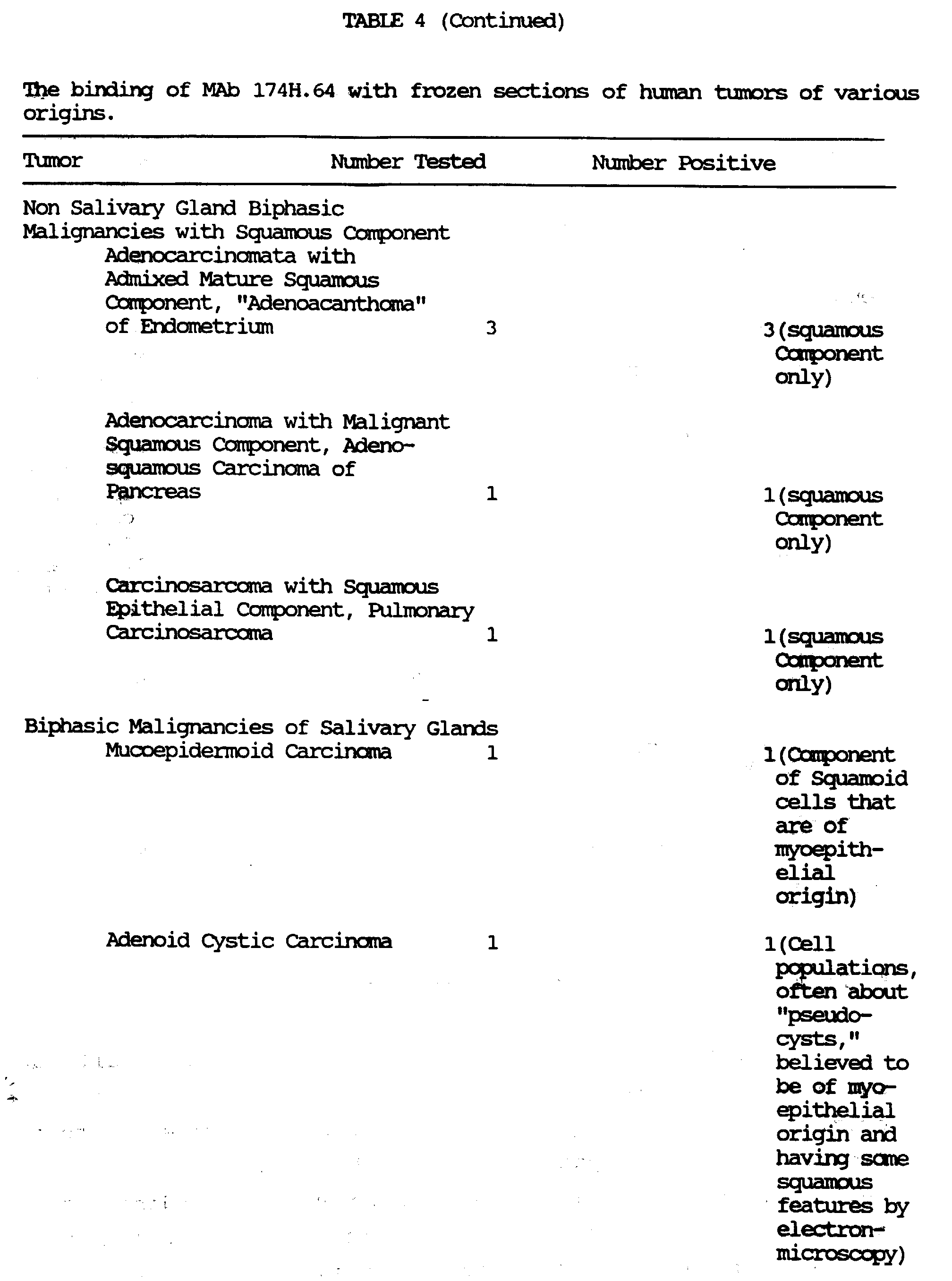

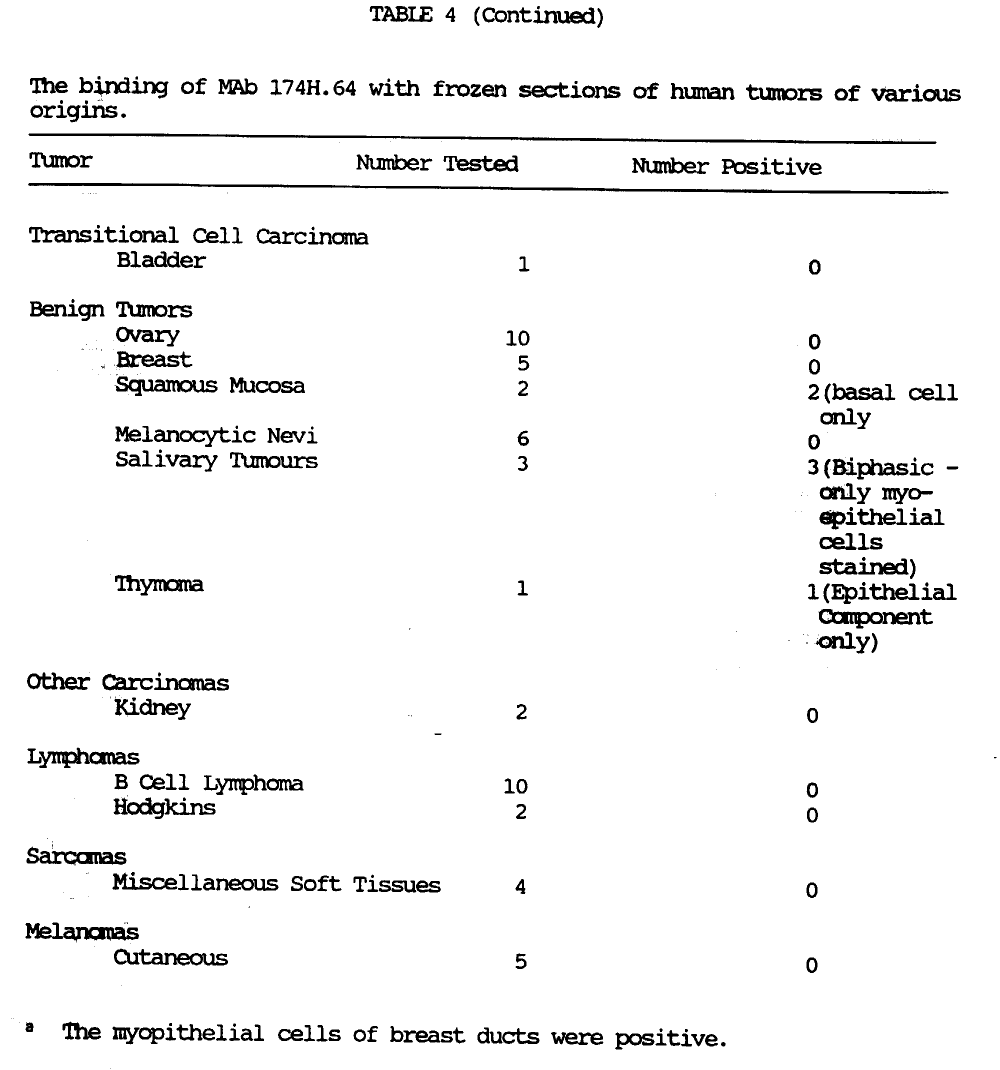

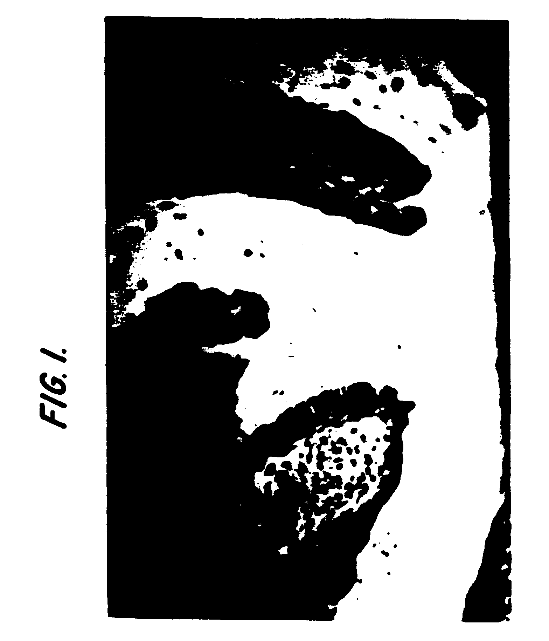

MAb 174H.64 showed selective binding with all human squamous cell carcinomas tested irrespective of the organ of origin, for a variety of other histological type of tumors. Figure 1 shows the pattern of MAb 174H.64 binding to squamous cell carcinoma tissues with preferential binding of the antibody to the peripheral layer of the tumor. The normal tissues adjacent to the tumor showed no binding with the antibody. Several spontaneously arising tumors from other mammals were also studied for their reactivity against MAb 174H.64 and the results are summarized in Table 5.

**TABLE 5**

| The binding of MAb 174H.64 with frozen section sof non-human tumors. | | |
|---|---|---|
| Tumor | Number Tested | Number Positive |
| Canine Tumors | | |
| Squamous/Basal Cell Carcinomas | 10 | 10 |
| Other carcinomas | 14 | 0 |
| Benign basal cell tumors | 4 | 4 |
| Papillomas^{a} | 4 | 4 |
| Perianal adenoma^{a} | 2 | 2 |
| Other benign epithelia tumors | 5 | 0 |
| Mesenchymal tumors | 45 | 0 |
| Bovine Squamous Cell Carcinoma | 4 | 4 |
| Feline Squamous Cell Carcinoma | 2 | 2 |

| | | |
|---|---|---|
| ^{a} In papillomas and perianal adenomas only the basal cell population showed staining with the antibody. | | |

Canine squamous and basal cell carcinoma tissues showed selective binding with the antibody while other carcinomas and tumors of mesenchymal origin did not show any reactivity. The reactivity of the antibody was also observed for bovine and feline squamous cell carcinomas. In the staining of a well-differentiated metastitic bovine lung squamous cell carcinoma, we observed increased antibody binding to the peripheral tumor cells. Among the benign epithelia tumors of dogs, basal cell tumors Showed homogeneous staining, whereas in papillomas and perianal adenomas, the staining was limited to basal cells only.

### Reactivity of MAb 174H.64 With Normal Tissues

Normal human tissues were studied for their reactivity with MAB 174.64 and the results are summarized in Table 6. Highly selective reactivity was observed for the basal cells of the stratified epithelium, thymic epithelium, and myoepithelial cells around breast ducts. No reactivity was observed for non-stratified epithelium (e.g., colon) or tissues of non-epithelial origin.

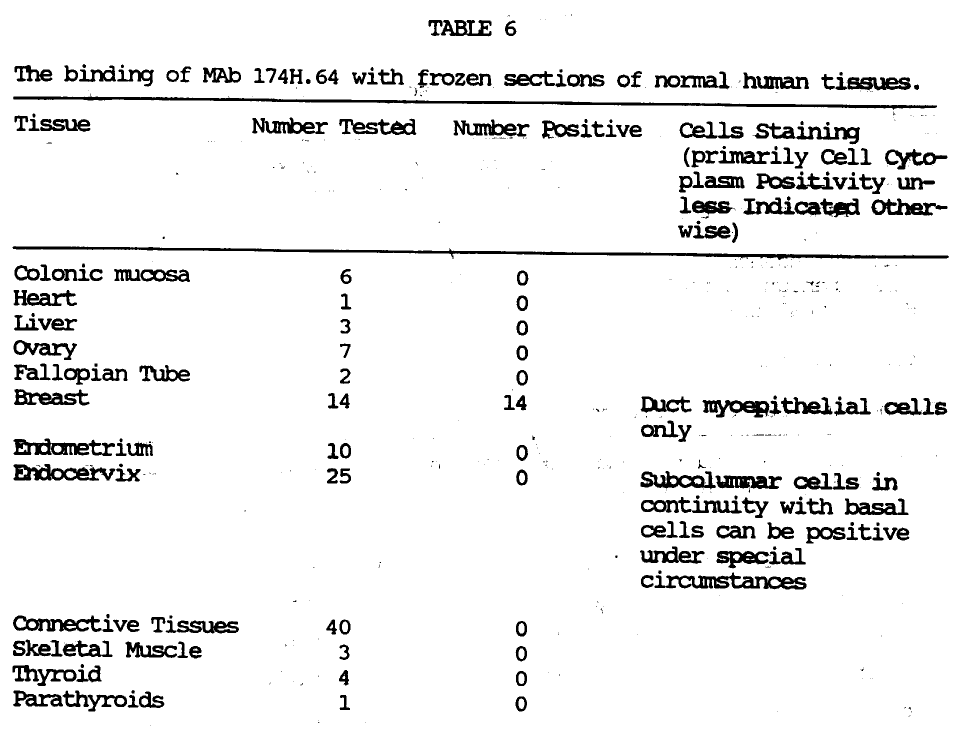

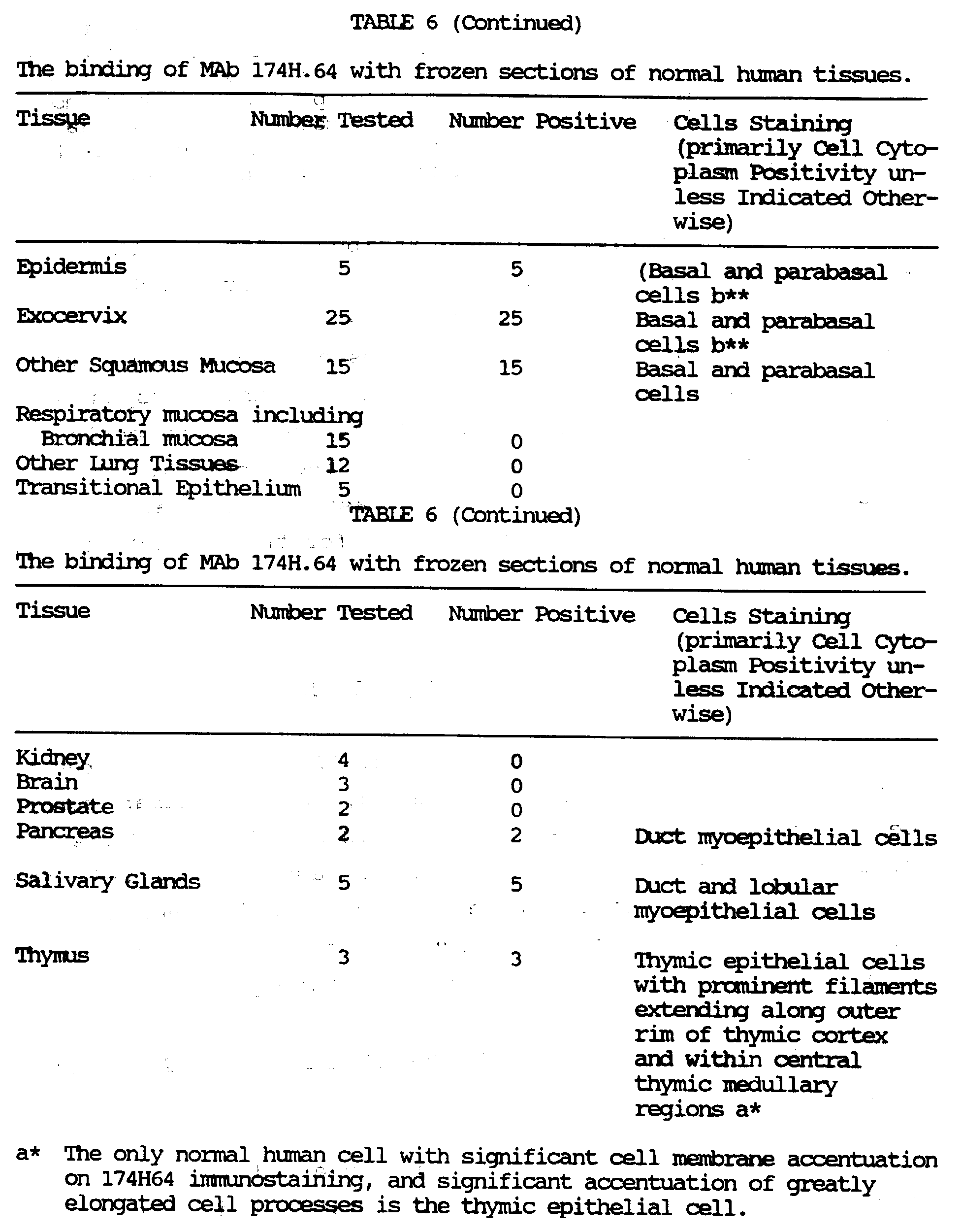

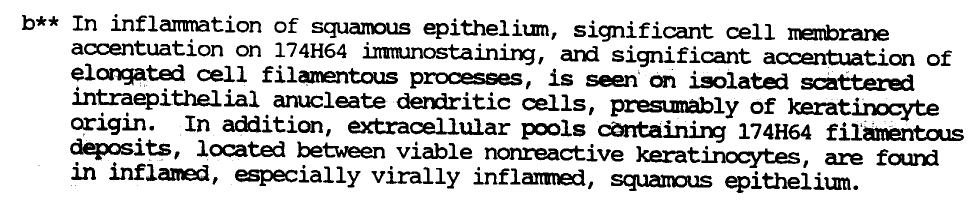

The reactivity of the antibody with the basal layer of the stratified epithelium was also demonstrated on bovine and mouse skin. A mouse keratinocyte suspension enriched in basal cells was prepared by trypsin dissociation of the neonatal mouse skin. A cell smear of this suspension on immunoperoxidase staining showed 30 to 50% of the cells to be positive for their reactivity with the antibody. A normal human keratinoyte culture having basal cell characteristics demonstrated all cells to be positive for their reactivity with the antibody.

### Reactivity of MAb 174H.64 With SCC Cell Lines

While as previously stated, MAb 174H.64 was reactive with all SCC tissue sections tested, several human squamous cell carcinoma cell lines (SCL-1, SIHAA, ME 180, C-33A) were tested for their reactivity with the antibody by cell smear immunoperoxidase staining and were found negative. In contrast, a murine metastatic lung squamous cell carcinoma cell line (KLN-205) was reactive with the antibody.

### Reactivity of MAb 174H.64 with Squamous Cell Carcinoma Animal Model

KLN-205 cells (10⁶) were injected i.v. into 8 week old DBA/2 male mice by a method standard in the art. The lungs, dissected from these mice after 21 days, showed extensive tumor colony formation. This tumor on immunoperoxidase staining showed strong reactivity with the antibody and preferential staining of the cells in the periphery of the tumor. Adjacent normal tissues as well as the lung tissue from a normal mouse did not show any binding with the antibody.

### Characteristics of the antigen

Protein extracts were prepared from a bovine metastatic lung squamous cell carcinoma tumor, a human lung squamous cell carcinoma tumor, a human ovarian adenocarcinoma tumor, and normal human epidermal keratinocytes grown in vitro as follows. The tissue samples or cell pellets were homogenized with 10 volumes of a Nonidet™ P-40 (NP-40, Sigma Chemical Company) extraction buffer (1% NP-40, 2 mM EDTA, 1 mM penyl methyl sulphonyl fluoride, 2 mM pepstatin 10 mM o-phenanthrolin, and 150 mM NaCl in 50 mM Tris HCl buffer pH 7.5) at 4°C for 30 minutes and then centrifuged at 100,000g for 1 hour. The residue was again extracted using 10 volumes of sodium dodecyl sulfate (SDS) extraction buffer (2% SDS and 10 mM 2mercaptoethanol in 10 mM Tris HCl buffer pH 7.5) at room temperature for 10 minutes, boiled at 100°C for 10 minutes, and then centrifuged at 10,000g for 15 minutes. The proteins in these extracts were separated on a discontinuous gradient SDS-poly acrylamide gel electrophoresis (4% stacking gel and a 4% to 15% gradient resolving gel) under standard reducing conditions. After electrophoresis, the gel was incubated in 200 mL of protein refolding buffer (4 M urea, 2 mM EDTA, 50 mM NaCl, 0.1 mM dithiothreitol in Tris HCl pH 7.5) for 18 hours with one change of buffer at room temperature and then equilibrated in transfer buffer (190 mM glycine in 25 mM Tris HCl pH 8.8) at 4°C for 1 hour. The proteins were electrophoretically transferred from the gel to a nitrocellulose membrane (Biorad) at 180 mA at constant current for 18 hours. The nitrocellulose sheets were removed, incubated in the blocking buffer containing 3% gelatin in PBS for 1 hour and washed twice in TPBS (PBS containing 0.05% Tween™ 20) for 5 minutes. They were then incubated with ¹²⁵I labelled antibodies (5 x 10⁵ cpm/mL) in antibody buffer (TPBS containing 1% gelatin) for 2 hours and washed 6 times in TPBS for 5 minutes each. The nitrocellulose sheets- were air dried and exposed to Xray film at -70°C. The immunoblots of the NP-40 extracts of the above tissues and the SDS extracts of adenocarcinoma showed no binding with ¹²⁵I labelled MAb 174H.64, while the immunoblots of the SDS extracts of bovine squamous cell carcinoma, human squamous cell carcinoma and human keratinocytes showed selective binding of ¹²⁵I labelled MAb 174H.64 with specific protein bands. The immunoreactive protein bands in keratinocyte SDS extract were of approximate molecular weight of 48 to 50kD (possibly a doublet) and 57kD. The bovine and human squamous cell carcinoma SDS extracts showed only one prominent band each with the approximate molecular weight of 48 to 50kD for the former and 57kD for the latter. A control antibody (155H.7) failed to bind with any of the above tumor extracts.

### Reactivity of MAb 174.64 with normal and premalignant epithelium of cervix

Frozen sections of normal and premalignant epithelium of cervix were examined for binding with MAb 174H.64. The results are summarized in Tables 7 and Table 8.

**TABLE 7**

| Reactivity of MAB 174H.64 with normal epithelium of cervix. | | |
|---|---|---|
| Epithelium | No positive/no tested | % positive |
| Mature stratified epithelium | | |
| Basal layer | 36/37 | 97.3 |
| Parabasal layer | 30/37 | 81.1 |

| Immature stratified epithelium | | |
|---|---|---|
| Basal layer | 51/70 | 72.9 |
| Parabasal layer | 41/70 | 58.5 |
| Subcolumnar epithelium | 8/16 | 50 |

**TABLE 8**

| Reactivity of MAb 174H.64 with premalignant lesions of cervix. | | |
|---|---|---|
| Preneoplastic lesion | No positive/no tested | % positive |
| Cervical Intrapithelial neoplasia (CIN) | | |
| Basal layer | 36/52 | 69.2 |
| Parabasal layer | 28/52 | 53.9 |
| Koilocytotic atypia | 16/16 | 100 |

In mature and immature squamous epithelium of the normal cervix, the staining was limited to basal and parabasal layers only. In the mature epithelium virtually 100% of the basal and parabasal layers were stained whereas in the immature epithelium the staining was limited to a range between 71 and 73%. In the subcolumnar epithelium in 70% of the cases staining could be observed for the reserve cells. These results suggest that the expression of this antigen is acquired during the process of maturation of the epithelium and is lost while the stem cells of the mature epithelium undergoes differentiation. The staining pattern of the reserve cells can reflect the proportion of cases where reserve cells are committed to become the stem cells of the stratified epithelium.

### Example 2: Immunotherapeutic Use of MAb 174H.64

MAb 174H.64, which selectively recognizes an epitope expressed on the proliferating cells of mammalian squamous carcinomas (Sq Ca), was covalently coupled to daunomycin (DM) by an acid sensitive linker and tested for its selective cytotoxicity for Sq Ca. A murine lung Sq Ca model for chemoimmunotherapy using MAb 174H.64-DM conjugates was developed. This model utilizes the KLN-205 Sq Ca cell line which metastasizes to the lungs following i.v. injection and shows a pattern of growth similar to those of spontaneous Sq Ca, that is, characterized by highly proliferative cells at the periphery of the tumor (reactive with 174H.64) with the keratinized differentiated cells toward the center (not reactive with 174H.64). MAb 174H.64-DM conjugates showed marked and specific cytotoxicity against KLN-205 cells both in vitro and following i.v. injection of the immunoconjugate in mice with established lung metastases. The conjugate was nearly as effective as DM alone when incubated in vitro with KLN-205 cells and much more effective in vivo than DM alone. Control immunoconjugates were ineffective. Finally, while the free 174H.64 MAb produced a significant increased time of survival of mice bearing KLN-205 metastases, a much greater survival was found with mice treated with the 174H.64-DM immunoconjugate with some mice apparently demonstrating long term survival (>100 days). We conclude that MAb 174H.64 may have potential therapeutic benefit against Sq Ca.

### Materials and Methods

Experimental animals: DBA/2 male mice, 8-12 weeks of age, were obtained from the Medical Sciences Animal Centre, University of Alberta.

Cell lines: A murine Sq Ca line, KLN-205 (Kaneko, et al., 1978), a murine leukemia cell line, SL2R5, Wolcsin, et al., Int. J. Cancer, 23:519 (1979), and a human myeloid leukemia line, HL60-2, Gallagher, et al., Blood, 54:713 (1979), were obtained from the American Type Culture Collection, Rockville, MD. All cell lines were maintained in RPMI 1640 medium supplemented with 10% FCS. KLN-205 is a murine lung Sq Ca cell line which metastasizes to the lung following i.v. injection. Kaneko, et al., Cancer Res., 28:2084 (1978). The resulting lung metastases reacted with MAb 174H.64, demonstrating selective reactivity with the growing cells in the periphery of each tumor. Samuel, et al., Cancer Res., 49:2465 (1989), Williams, et al., J. Nat. Canc. Inst. 51:1513 (1973).

Preparation of immunoconjugates: Immunoconjugates have been made in which the cytotoxic component was daunomycin (DM) attached to the target-specific conjugand via the acid-sensitive cis-aconityl spacer (CA). Shen, et al., Biochem. Biophys. Res. Comm., 102:1048 (1981). These daunomycin immunoconjugates showed high specific cytotoxicity on target cells and could be used for purging tumor cells and functional T cells from bone marrow. The present study was designed to test the potential of MAb 174H.64-daunomycin immunoconjugates to target therapy to KLN-205 Sq Ca lung tumors. Immunoconjugates were prepared as described, Diener, et al., in Antibody-Mediated Delivery System, p. 1 (Marcel Dekker, Inc., N.Y. 1988) and Xie, et al., Transplantation, 44:770 (1987), according to the method of Shen, et al.(1981). MAbs were obtained from ascites fluids which were subjected to protein precipitation with 40% (NH₄)₂SO₄ followed by dialysis against phosphate buffered saline at 4°C for 24 hours. This protein fraction was further purified by affinity chromatography on a protein A-sepharose column (Pharmacia). Daunomycin and cis-aconitic anhydride were purchased from Sigma Chemical Co., St. Louis, MO. Purified monoclonal antibody 174H.64 was, obtained as described above. MAb anti-Thyl.2 was obtained from NEN and MAb 86H.1 which reacts against human myeloid cells, See Janowska-Wieczorek, et al., in Leukocyte Typing II, Vol. 3, Human Myeloid and Hematopoietic Cells, p. 171 (Springer Verlag, N.Y. 1986), including HL-60 cells, was made in our laboratories.

Treatment of cells with cytotoxic immunoconjugates or other agents: Cells in phosphate-buffered saline (PBS) were treated with the immunoconjugates or other agents for 2 1/2 hours on ice. Thereafter the cells were washed 3 times and injected intravenously into recipient mice, or incubated for 24 hours at 37°C after which ³H-thymidine incorporation by the cells was measured.

³H-thymidine incorporation: Cell cultures were pulsed for four hours with 0.5 µCi/well of ³H-thymidine (specific activity 2.0 µCi/mmol) (NEN). Cells were harvested onto glass fibers with a Titertek semi-automated multiple sample collector (Flow Laboratories, Mississauga, Ontario). Thymidine incorporation was determined by liquid scintillation spectrometry.

Squamous carcinoma animal model: As described previously (Samuel, et al., 1989) using KLN-205 cells (Kaneko, et al., 1978).

¹²⁵I-IUDR uptake: Mice were injected with 2 µCi/each of ¹²⁵I-IUDR (Edmonton Radiopharmaceutical Centre, Edmonton, Alberta, Canada) and 4 hours later, the lungs were removed, cut into small pieces, washed with 10% trichloroacetic acid (TCA) 3 times for three days and assessed for radioactivity content using a gamma-counter.

Statistical analysis: Most data were assessed by Student's t-test. Survival data were assessed by modified Wilcoxon.

### Results

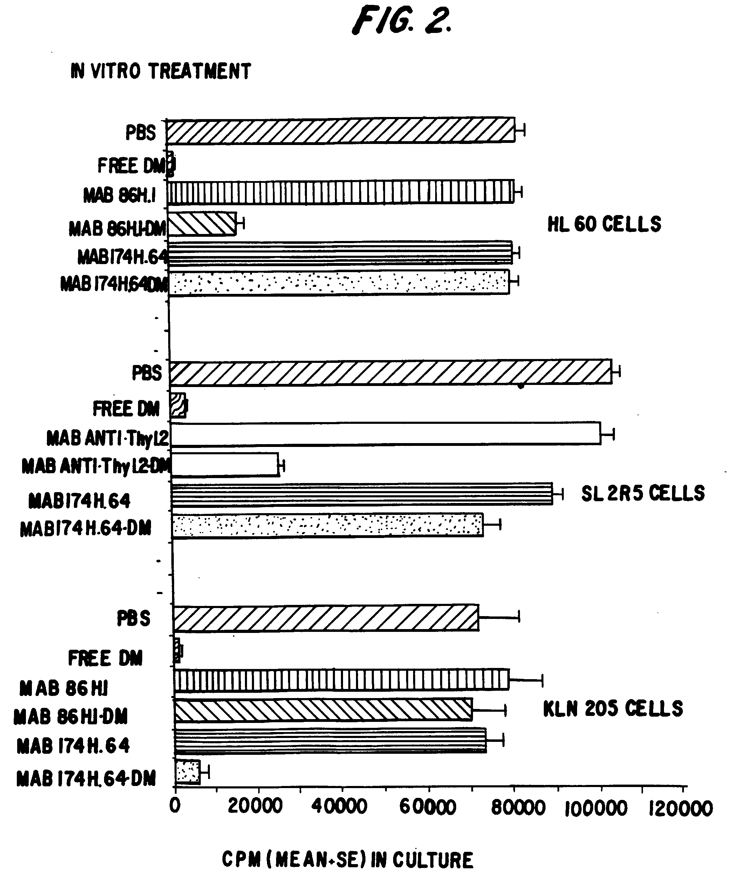

Inhibition of in vitro proliferation of KLN-205 cells by MAb 174H.64-DM: We have previously described the selective reactivity of MAb 174H.64 with all mammalian Sq Ca tested including the KLN-205 murine lung squamous carcinoma cell line (Samuel, et al., 1989). To investigate the specific cytotoxicity of the immunoconjugate, the KLN-205 cell line, SL2R5 cell line and HL60-2 cell line were treated on ice for 2 hours with MAb 174H.64-DM, MAb 86H.1-DM, MAb anti Thyl.2-DM, free DM, MAb 174H.64, MAb anti-Thyl.2 or MAb 86H.1, and following washing, the cells were subsequently cultured for another 24 hours. Fig. 2 demonstrates the specificity of cell killing of the different antibodies in comparison with the daunomycin alone. While the HL-60 cells and SL2R5 cells were each inhibited by their respective MAbs, only KLN-205 cells were inhibited by the MAb 174H.64-DM immunoconjugate. It is noteworthy that the inhibition in vitro of the proliferation of KLN-205 cells by the MAb 174H.64-DM immunoconjugate was nearly as effective as the daunomycin alone.

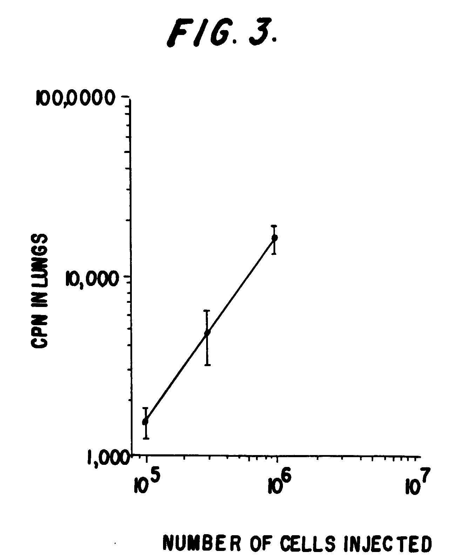

In vitro treatment of KLN-205 cells with MAb 174H.64-DM inhibits tumor cell proliferation in the lungs of i.v. injected mice: For this experiment we employed the ¹²⁵I-IUDR uptake assay as a measure of RLN-205 tumor cell proliferation in the lungs. We first established that the uptake of ¹²⁵I-IUDR in the lungs is directly proportional to the number of i.v. injected KLN-205 cells at least over the range of 10⁵ to 10⁶ cells (Fig. 3).

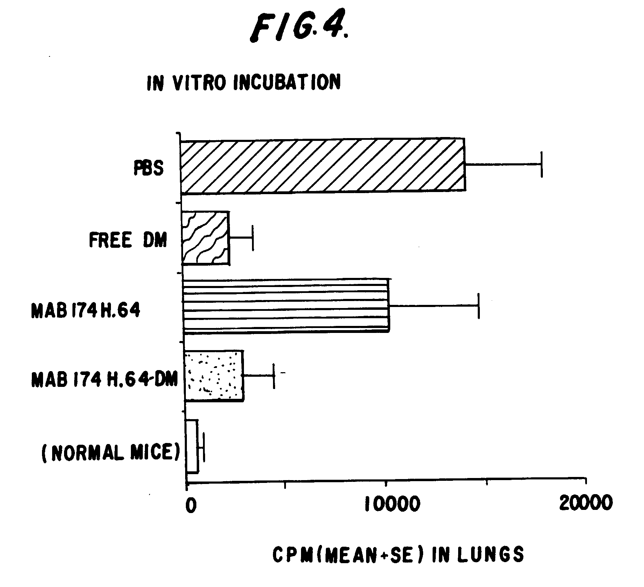

KLN-205 cells were then treated on ice for 2 hours with the MAb 174H.64-DM immunoconjugate or with various controls, including PBS, MAb 174H.64 alone or free DM. Thereafter cells were washed three times and injected into DBA/2 mice (10⁶ cells/mouse). On the 23rd day after injection, tumor growth in the lung was monitored using ¹²⁵I-IUDR uptake. Results in Fig. 4 show that the MAb 174H.64-DM pretreatment significantly reduced the growth of lung tumors approximately 85% and was as effective as incubation of the cells with DM alone (Fig. 4).

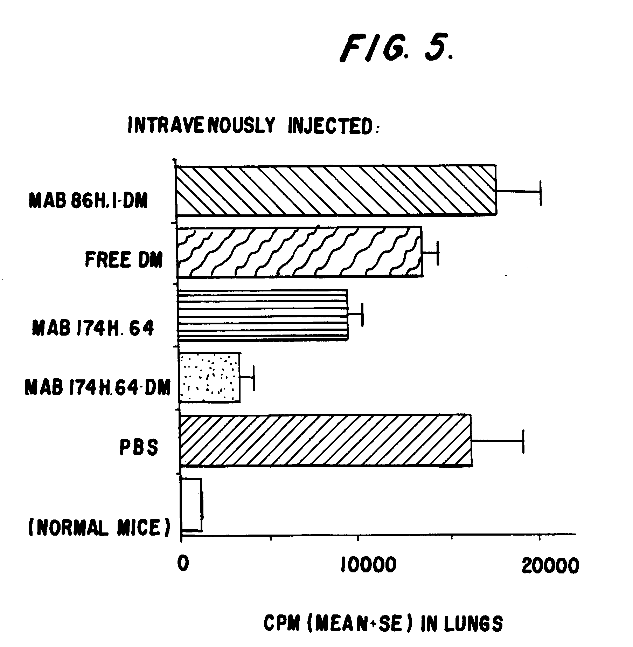

I.V. injected MAb 174H.64-DM inhibits the growth of established lung metastatic KLN-205 cells in vivo: The purpose of this experiment was to investigate the specific cytotoxicity of MAb 174H.64-DM in vivo. 10⁶ KLN-205 cells were injected i.v. into DBA/2 mice. Three days later MAb 174H.64-DM was injected i.v. every second day for 10 days (5 injections). On the 23rd day after injection of the tumor cells, tumor growth was measured by assessing ¹²⁵I-IUDR uptake in the lungs. The results in Fig. 5 show that. MAb 174H.64-DM significantly (p<0.001) reduced ¹²⁵I-IUDR uptake by about 80% while MAb 86H.1-DM had no significant effect. Daunomycin alone had a slight, but insignificant effect but MAb 174H.64 alone reduced the tumor cell proliferation approximately 42% (P<0.001).

Survival of mice receiving MAb 174H.64-DM Therapy: The above experiment was repeated but instead of sacrificing the mice to monitor the growth of KLN--205 cells in the lungs, the mice were simply monitored for survival. The results in Fig. 2 show that MAb 174H.64-DM treated mice survived significantly longer (p<0.001) than untreated or antibody treated mice. MAb 174H.64 alone also improved the survival of tumor bearing mice (P<0.001).

### Discussion

The present study was designed to test the hypothesis that a MAb directed against the proliferative (STEM) cell compartment of squamous carcinomas has therapeutic potential. We have used a novel MAb, 174H.64 which appears to detect a unique epitope on cytoskeletal proteins which may serve as a marker for the stem cell population in normal stratified squamous epithelia and squamous carcinomas (Samuel, et al., 1981). MAb 174H.64 reacts with all Sq Ca tested regardless of histological origin (Idem.) as well as with all Sq Ca of all mammals tested, including those of humans (>62 tested), dogs (10 tested), cattle (40 tested) and cats (2 tested). In the present study we took advantage of its reactivity with a murine metastatic lung Sq Ca, KLN-205, to develop an animal model for chemoimmunotherapy with a daunomycin conjugate of MAb 174H.64 (MAb 174H.64-DM). Previous studies using the KLN-205 cell line have shown that the tumor periphery of the lung metastatic nodules is composed of highly proliferative cells which are the progenitors of the more differentiated cells in the central portion of the tumor. We have shown that MAb 174H.64 selectively stains the peripheral layer of KLN-205 lung tumors as well as human and bovine Sq Ca. This provided the rationale for using the KLN-205 model to attempt to selectively target therapy to the "stem" cells of the tumor as it might be expected that the internal, more differentiated cells are no longer capable of neoplastic proliferation.

We chose daunomycin as the cytotoxic component of the immunoconjugate because of our extensive experience with this drug. Diener, et al., Science, 231:148 (1986); Diener, et al., in Cellular Basis of Immune Modulation, p. 509 (Alan R. Liss, Inc., NY 1988); Diener, et al. (1988)(Marcel Dekker); Xie, et al. (1987). We have previously shown that an immunoconjugate of anti-Thyl.2 MAb and daunomycin possesses high specific cytotoxicity for target cells and could be used for the specific purging of Thy1.2+ tumor cells and murine functional T cells from the bone marrow. We have discovered that an immunoconjugate of MAb 174H.64 with daunomycin also produces marked, specific inhibition of the growth of KLN-205 murine squamous carcinoma both in vitro and in vivo when compared to irrelevant conjugates, free monoclonal antibodies, or free daunomycin.

Our first experiments were designed to demonstrate the specificity of the immunoconjugate killing in vitro. For this we conducted a criss-cross experiment with three cell lines and three immunoconjugates which, based on immunofluorescent and/or immunoenzyme testing should be specific for their specific cell lines. Exquisite specificity and excellent killing was noted in each case: MAb86H.1-DM killed only HL-60 cells, MAb anti-Thy1.2-DM killed only SL2-R5 cells and MAb 174H.64 killed only KLN-205 cells. Interestingly, the killing of KLN-205 cells by the 174H.64 immunoconjugate was nearly as effective as the free daunomycin added to the culture.

An extension of the in vitro experiment was then performed whereby the KLN-205 cells were incubated in vitro with the immunoconjugate, free antibody or daunomycin, washed, injected i.v., and the metastatic growth in the lungs monitored. In this case a marked reduction of the metastatic growth of the KLN-205 cells in the lungs was achieved by preincubation with the MAb 174H.64-DM conjugate. Again the reduction achieved with the immunoconjugate was about the same as that achieved with free daunomycin. In vitro incubation with the unconjugated MAb 174H.64 caused a slight but statistically non-significant reduction in growth of KLN-205 cells in the lungs.

The next two experiments were designed to directly test the in vivo therapeutic potential of the MAb-174H.64-DM immunoconjugate. Immunotherapy was started three days following the i.v. injection of 10⁶ KLN-205 cells, at a time when the lung metastatic cells should have been well established (Kaneko, et al., 1981; Williams, et al., 1973). Five therapeutic injections of MAb-174H.64-DM were given i.v. every second day for ten days. Growth of the KLN-205 cells in the lungs was monitored at day 23 by using the ¹²⁵I-IUDR uptake assay and survival was monitored for >100 days. The two estimates of the therapeutic potential of the various compounds gave very similar results. Mice injected with MAb 86H.1-DM showed no therapeutic benefit and showed similar tumor growth and survival as those injected with PBS. Daunomycin alone gave a slight reduction of growth and a slight increase in survival. An even greater therapeutic benefit was achieved with unconjugated MAb 174H.64. However by far the best therapy was achieved following i.v. injection with the MAb 174H.64-DM immunoconjugate where an >80% reduction in tumor cell growth in the lungs and a prolonged survival was achieved. Greatly prolonged survival was achieved in 20% (5 mice) of the mice which survived >100 days. The therapeutic benefit of the 174H.64 immunoconjugate is even more impressive if one considers that the therapeutic regimen used in the present experiments may not be an optimal one.

The efficacy of MAb 174H.64-DM probably depends on the specificity of its binding to the target cell surface and the accumulation of the drug inside the target cells. Broxterman, et al. Biochem. Pharmacol., 37:2389 (1988); Dillman, et al., Cancer Res., 48:6097 (1988) ; Pimm, et al., Cancer Immunol. Immunother., 27:267 (1988). Pimm and coworkers have studied human tumors growing in nude mice injected with daunomycin immunoconjugates. They found that the tumors showed localization of both the drug and antibody and at the time of analysis (3 days after injection), the tumor levels of daunomycin were over 100 times those seen in mice injected with free daunomycin Following endocytosis of the immunoconjugate, the potentially cytotoxic component appears -to regain full pharmacologic activity upon release from the acid-sensitive spacer in lysosomes (Dillman, et al. 1988). Those results are consistent with our observations that at the same daunomycin concentration the immunoconjugate MAb 174H.64-DM showed a marked suppression of the growth of KLN-205 cells in vivo while free daunomycin did not, although the free daunomycin showed the same or better cytotoxic potency as the immunoconjugate following in vitro incubation.

Gallego, et al. (1984) investigated four different linkage groups for coupling daunomycin to antibody. They found that conjugates made with a cis-aconityl linkage, which was the same one used in the present work, displayed the greatest selective cytotoxicity against tumor cells. Our results appear to substantiate the efficacy of daunomycin immunoconjugate made with cis-aconityl linkage groups.

MAb 174H.64 appears to detect a novel epitope expressed on certain cytoskeletal proteins expressed only in the stem cell populations of normal and neoplastic stratified squamous epithelium. The present experiments as well as our previous observations suggest that the epitope detected by MAb 174H.64 is also expressed on the surface of the KLN-205 cells. Other cell surface determinants have been reported for some cytoskeletal proteins, see Diaz, et al., J. Invest. Dermatol., 89:287 (1987); Zimmerman, et al., J. Cell. Biol., 95:237a (1982). As the 174H.64 epitope may also be present on the surface of the basal cells of stratified squamous epithelium some skin toxicity might have been expected in the present experiments. However, we have never seen any evidence of any skin lesions nor any other obvious gross pathology in over 100 mice which have received multiple injections of either the free 174H.64 MAb or the MAb 174H.64-DM conjugate including our long term (>100 day) survivors.

Based on our studies in mice we conclude that MAb 174H.64 may have promising therapeutic potential in Patients with squamous cell carcinoma. In our preliminary radioimmunoimaging studies with radiolabeled MAb 174H.64 excellent in vivo localization of this MAb in human squamous cancer was observed.

### Example 3: Immunoimaging Using MAb 174H.64

### Clinical Utility of MAb 174H.64

To test the clinical usefulness of radiolabeled MAb 174.H.64 as an in vivo tumor localizing agent the purified whole antibody was labeled with Technetium-99m and injected in a number of patients bearing squamous cell carcinomas.

The labeling procedure was as follows:

A mixture of Sodium Potassium Tartarate (0.282 g) and Potassium Phthalate (0.817 g) was dissolved in 100 ml -sterile water for injection in a pyrogen-free beaker with constant stirring (the "buffer solution"). The solution was purged with dry, particle-free nitrogen for 30 min. The nitrogen source was previously passed through a nitrogen purifier to remove trace amounts of oxygen and then filtered through a 0.02µ filter to remove any residual particulate contaminants. A stannous chloride solution was then prepared by dissolving 1.896 g of anhydrous stannous chloride in 20 ml of concentrated hydrochloric acid to make a 0.5M SnCl₂ solution. One milliliter of the stannous solution was added immediately to the previously prepared buffer solution and the pH of this final mixture was adjusted carefully to 5.6± 0.05 with 10 N sodium hydroxide solution. The mixture was purged with nitrogen gas and filtered through a 0.22µ filter. This is called the reducing solution. One milliliter of MAb 174H.64 solution in phosphate, buffer was then mixed with 0.67 ml of filtered reducing solution and the final MAb concentration was adjusted such that the mixture contained 1 mg antibody/ml. After mixing, the vial containing the antibody and reducing solution was purged with nitrogen for several minutes and kept under a nitrogen atmosphere at room temperature for 21 hours. After the incubation period, the treated antibody solution was filtered through a 0.22µ filter. The latter filtrate was assayed for its protein content, for pyrogens, for purity (using size exclusion column chromatography) and for immunoreactivity. No differences were observed between the original untreated whole antibody and the derivatized one either in terms of immunoreactivity or molecular weight. The solution of prepared antibody was then frozen until ready for use clinically. At the time of use, the vial containing the frozen reduced antibody is thawed at room temperature and the required amount of TC-^{99m} sodium pertechnectate solution (up to 50 mCi) were added and the mixture allowed to incubate for 1 hour at room temperature. At the end of the incubation period, a sample of the mixture was tested for any unbound TC-^{99m} by thick layer chromatography using acetone as a developing solvent. Radiolabeling efficiency was greater than 95%.

### Clinical Results

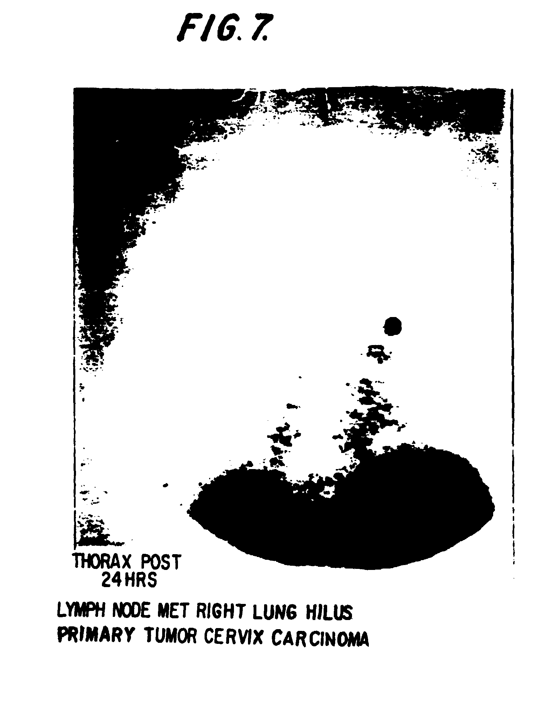

A clinical study involving 11 patients with metastatic squamous cell carcinoma of the head and neck, cervix, and lung was conducted to evaluate the uptake of ^{99m}Tc-labeled MAb 174H.64 after intravenous injection into these patients. An antibody dose of 2 mg (about 50 mCi) was diluted with 100 ml normal saline and infused over 30 minutes. Any abnormal uptake was measured by planar and single photon emission computerized tomography at various intervals after the injection. Results obtained within 24 hours indicated that two patients showed an apparent negative uptake while 9 patients were shown to be truly positive. Figure 7 illustrates such a scan observed for a patient with cervical squamous cell carcinoma whose tumor had metastasized to the upper chest region. It demonstrates the uptake of the antibody in a lesion estimated at 2-4 mm in diameter (see Fig. 7)

We found it to be unusual that the pharmacokinetics of a labeled whole antibody is indeed similar to the known behaviour of a fragment. Blood clearance was expeditious and uptake could be seen as early as 3 hours after injection. Analysis of the radioactivity in the patient's urine has demonstrated that the secreted radioactivity was not due to free pertechnectate, but rather due to bound radioactivity.

## Claims

1. A monoclonal antibody or a fragment hereof, which essentially only binds to squamous cell carcinomas and basal and parabasal layers of stratified squamous epithelium, said antibody or fragment hereof binding to an antigen associated with the squamous cell carcinomas, which antigen is also bound by monoclonal antibody 174H.64 which is secreted by a hybridoma cell line having ATCC deposit number HB 10105, or a reclone hereof, or to an epitope of said antigen.

2. A monoclonal antibody according to claim 1, or a fragment hereof, where said antibody or fragment, and antibody 174H.64 both bind to the same epitope of said antigen.

3. A monoclonal antibody according to claim 1 or 2, or a fragment hereof, which binds preferentially to antigens associated with squamous cell carcinomas and basal and parabasal layers of stratified squamous epithelium and which, in squamous cell carcinomas, reacts most strongly with the proliferative compartment hereof.

4. A monoclonal antibody according to claim 1 or 2, or a fragment hereof, selected from 174H.64 antibody or a fragment hereof and a monoclonal antibody or a fragment hereof which binds to an antigen associated with the squamous cell carcinomas, which antigen is also bound by monoclonal antibody 174H.64, or to an epitope of said antigen.

5. A monoclonal antibody according to claim 1 or 2, or a fragment hereof, where said epitope is not bound by monoclonal antibody 17.13.

6. A monoclonal antibody according to claim 1 or 2, or a fragment hereof, which binds to a squamous cell carcinoma-associated antigen which has the same antigen specificity and binding characteristics as monoclonal antibody 174H.64, or which is monoclonal antibody 174H.64.

7. A monoclonal antibody according to claim 1 or 2, or a fragment hereof, derived from an antibody which is secreted by a hybridoma having ATCC deposit number HB 10105 or a reclone thereof.

8. An antibody according to any of claims 1-7, or a fragment hereof, wherein the antigens bound are cytoskeletal proteins having molecular weights of approximately 48 to 50kD and 57kD.

9. An antibody according to any of claims 1 and 4-7, or a fragment hereof, wherein said antibody or fragment hereof reacts with a proliferative compartment of said human squamous cell carcinoma.

10. An antibody according to any of claims 1-9, or a fragment hereof, which selectively binds with human squamous cell carcinomas irrespective of the tissue of origin hereof.

11. An antibody according to any of claims 1-10, or a fragment hereof, which reacts with premalignant lesions of the squamous epithelium.

12. An antibody according to any of claims 1-11, or a fragment hereof, said antibody or fragment being labelled or in solubilized form.

13. Use of a radiolabelled monoclonal antibody or a fragment hereof according to any of claims 1-11 in the manufacture of a composition for determining a location of a squamous cell carcinoma in a patient by immunoimaging of said radiolabelled monoclonal antibody or fragment.

14. Use of a therapeutically effective amount of a monoclonal antibody or fragment hereof according to any of claims 1-11, said monoclonal antibody or fragment optionally being conjugated with a cytotoxic agent but retaining its specificity, in the manufacture of a composition for treating squamous cell carcinoma in a patient.

15. A method for detecting the presence of a squamous cell carcinoma associated antigen in a sample which comprises incubating a monoclonal antibody or fragment hereof according to any of claims 1-12 with said sample for a period of time sufficient to permit at least some of the antibody to bind to said antigen and detecting the bound antibody.

16. A process of isolating antigens binding to an antibody or a fragment hereof according to any of claims 1-11, comprising immobilizing the antibody or fragment hereof on a solid support, contacting said solid support with a biological sample, separating unbound impurities from the solid support, and eluting the antigen in a purified form from the immobilized antibody or fragment.

17. A method of diagnosing squamous cell carcinoma which comprises detection or quantification of the in vitro binding reaction of the antibody or fragment according to any of claims 1-12 and antigens associated with squamous cell carcinoma which are bound by the antibody or its fragment.

18. A method of detecting a premalignant lesion, such as cervical intraepithelial neoplasia or kilocytotic atypia, of squamous epithelium in a tissue sample which comprises reacting the sample with an antibody or a fragment according to any of claims 1-11 and determining whether binding occurs.

19. A therapeutic agent comprising an antibody or a fragment according to any of claims 1-10, which said antibody or fragment being conjugated with a cytotoxic agent such as daunomycin, said antibody or fragment essentially retaining its specificity.

## Patentansprüche

1. Monoklonaler Antikörper oder Fragment davon, welcher im wesentlichen nur an Schuppenzell-Carcinoma und Basal- und Parabasalschichten von vielschichtigen Schuppen-Epithel bindet, wobei der Antikörper oder das Fragment davon an ein mit dem Schuppenzell-Carcinoma assoziiertes Antigen bindet, wobei das Antigen ebenfalls von dem monoklonalen Antikörper 174H.64 gebunden wird, der von einer Hybridom-Zelllinie mit der ATCC-Hinterlegungsnummer HB. 10105 oder einem Reclon davon ausgeschieden wird, oder an ein Epitop des Antigens.

2. Monoklonaler Antikörper nach Anspruch 1 oder Fragment davon, worin sowohl der Antikörper oder das Fragment als auch der Antikörper 174H.64 an dasselbe Epitop des Antigens bindet.

3. Monoklonaler Antikörper nach Anspruch 1 oder 2 oder Fragment davon, der vorzugsweise an mit Schuppenzell-Carcinoma und Basal- und Parabasalschichten von vielschichtigem Schuppen-Epithel assoziierten Antigene bindet, und der bei Schuppenzell-Carcinoma am stärksten mit dem proliferativen Kompartment davon reagiert.

4. Monoklonaler Antikörper nach Anspruch 1 oder 2 oder Fragment davon, ausgewählt aus 174H.64-Antikörper oder einem Fragment davon, und einem monoklonalen Antikörper oder einem Fragment davon, der an ein mit Schuppenzell-Carcinoma assoziiertes Antigen bindet, wobei das Antigen ebenfalls von monoklonalem Antikörper 174H.64 gebunden wird, oder an ein Epitop des Antigens.

5. Monoklonaler Antikörper nach Anspruch 1 oder 2 oder ein Fragment davon, wobei das Epitop von dem monoklonalen Antikörper 17.13 nicht gebunden wird.

6. Monoklonaler Antikörper nach Anspruch 1 oder 2 oder ein Fragment davon, der an ein mit Schuppenzell-Carcinoma assoziiertes Antigen bindet, welches die gleiche Antigen-Spezifität und Bindungscharakteristik wie der monoklonale Antikörper 174H.64 hat, oder welches der monoklonale Antikörper 174H.64 ist.

7. Monoklonaler Antikörper nach Anspruch 1 oder 2 oder ein Fragment davon, abgeleitet von einem Antikörper, der durch ein Hybridom mit der ATCC-Hinterlegungsnummer HB 10105 oder einem Reclon davon ausgeschieden wird.

8. Antikörper nach einem der Ansprüche 1 bis 7 oder ein Fragment davon, worin die gebundenen Antigene Cytoskelett-Proteine mit Molekulargewichten von ca. 48 bis 50kD und 57kD sind.

9. Antikörper nach einem der Ansprüche 1 und 4 bis 7 oder einem Fragment davon, worin der Antikörper oder das Fragment davon mit einem proliferativen Kompartment des humanen Schuppenzell-Carcinoma reagiert.

10. Antikörper nach einem der Ansprüche 1 bis 9 oder einem Fragment davon, welches selektiv mit humanen Schuppenzell-Carcinoma unabhängig von dem Ursprungsgewebe reagiert.

11. Antikörper nach einem der Ansprüche 1 bis 10 oder Fragment davon, welches mit premalignen Verletzungen des Schuppen-Epithel reagiert.

12. Antikörper nach einem der Ansprüche 1 bis 11 oder ein Fragment davon, wobei der Antikörper oder das Fragment markiert ist, oder in solubilisierter Form vorliegt.

13. Verwendung eines radiomarkierten monoklonalen Antikörpers oder eines Fragments davon nach einem der Ansprüche 1 bis 11 bei der Herstellung einer Zusammensetzung zur Ortsbestimmung eines Schuppenzell-Carcinoms in einem Patienten durch Immun-Bildgebung des radiomarkierten monoklonalen Antikörpers oder Fragments.

14. Verwendung einer therapeutisch wirksamen Menge eines monoklonalen Antikörpers oder eines Fragments davon nach einem der Ansprüche 1 bis 11, wobei der monoklonale Antikörper oder das Fragment wahlweise mit einem cytotoxischen Mittel konjugiert ist, jedoch seine Spezifität bei der Herstellung einer Zusammensetzung zur Behandlung von Schuppenzell-Carcinoma in einem Patienten beibehält.

15. Verfahren zum Nachweis des Auftretens von Schuppenzell-Carcinoma assoziiertem Antigen in einer Probe, welche das Inkubieren eines monoklonalen Antikörpers oder Fragment davon nach einem der Ansprüche 1 bis 12 mit der Probe für einen Zeitraum umfaßt, der ausreicht, um zu ermöglichen, daß mindestens einiges vom Antikörper an das Antigen bindet, und Nachweis des gebundenen Antikörpers.

16. Verfahren zur Isolierung von Antigenen, die an einen Antikörper oder an ein Fragment davon nach einem der Ansprüche 1 bis 11 binden, umfassend das Immobilisieren des Antikörpers oder Fragments davon auf einen festen Träger, In-Kontaktbringen des festen Trägers mit einer biologischen Probe, Abtrennen von ungebundenen Verunreinigungen vom festen Träger und Eluieren des Antigens in gereinigter Form von dem immobilisierten Antikörper oder Fragment.

17. Verfahren zur Diagnose von Schuppenzell-Carcinoma, welches den Nachweis oder die Quantifizierung der in vitro-Bindungsreaktion des Antikörpers oder des Fragments nach einem der Ansprüche 1 bis 12 und der Antigene, die mit Schuppenzell-Carcinoma assoziiert sind, die durch den Antikörper oder ein Fragment davon gebunden werden, umfaßt.

18. Verfahren zum Nachweis von premalignen Verletzungen, wie cervikaler intraepithelialer Neoplasie oder kilocytotischer Atypie, von Schuppenzell-Epithel in einer Gewebeprobe, welches das Reagieren der Probe mit einem Antikörper oder einem Fragment davon nach einem der Ansprüche 1 bis 11 und Bestimmen, ob Bindung auftritt, umfaßt.

19. Therapeutisches Mittel, umfassend einen Antikörper oder ein Fragment davon nach einem der Ansprüche 1 bis 10, wobei der Antikörper oder das Fragment mit einem cytotoxischen Mittel, wie Daunomycin, konjugiert ist, wobei der Antikörper oder das Fragment im wesentlichen seine Spezifizität beibehält.

## Revendications

1. Anticorps monoclonal ou un de ses fragments, qui ne se lie essentiellement qu'aux carcinomes de cellules squameuses et aux couches basales et parabasales de l'épithélium pavimenteux stratifié, ledit anticorps ou son fragment se liant à un antigène associé aux carcinomes de cellules squameuses, cet antigène étant également lié par l'anticorps monoclonal 174H.64 qui est sécrété par une lignée cellulaire d'hybridome ayant le numéro de dépôt ATCC HB 10105, ou un de ses reclones, ou sur un épitope dudit antigène.

2. Anticorps monoclonal selon la revendication 1, ou un de ses fragments, ledit anticorps ou fragment, et l'anticorps 174H.64 se liant tous deux au même épitope dudit antigène.

3. Anticorps monodonal selon la revendication 1 ou 2, ou un de ses fragments, qui se lie de façon préférentielle aux antigènes associés aux carcinomes de cellules squameuses et aux couches basales et parabasales de l'épithélium pavimenteux stratifié et qui, dans les carcinomes de cellules squameuses, réagissent très fortement avec leur compartiment proliférant.

4. Anticorps monoclonal selon la revendication 1 ou 2, ou un de ses fragments, choisi parmi l'anticorps 174H.64 ou un de ses fragments et un anticorps monoclonal, ou un de ses fragments, qui se lie à un antigène associé aux carcinomes de cellules squameuses, cet antigène étant également lié par l'anticorps monoclonal 174H.64, ou à un épitope dudit antigène.

5. Anticorps monoclonal selon la revendication 1 ou 2, ou un de ses fragments, ledit épitope n'étant pas lié par l'anticorps monoclonal 17.13.

6. Anticorps monoclonal selon la revendication 1 ou 2, ou un de ses fragments, qui se lie à l'antigène associé au carcinome des cellules squameuses qui a la même spécificité antigénique et les mêmes caractéristiques de liaison que l'anticorps monoclonal 174H.64, ou qui est l'anticorps monoclonal 174H.64.

7. Anticorps monoclonal selon la revendication 1 ou 2, ou un de ses fragments, dérivé d'un anticorps qui est sécrété par un hybridome ayant le numéro de dépôt ATCC HB 10105 ou un de ses reclones.

8. Anticorps selon l'une quelconque des revendications 1-7, ou un de ses fragments, dans lequel les antigènes liés sont des protéines cytosquelettiques ayant des poids moléculaires d'environ 48 à 50 kD et 57 kD.

9. Anticorps selon l'une quelconque des revendications 1 et 4-7, ou un de ses fragments, ledit anticorps ou son fragment réagissant avec un compartiment prolifératif dudit carcinome de cellules squameuses.

10. Anticorps selon l'une quelconque des revendication 1-9, ou un de ses fragments, qui se lie de façon sélective avec des carcinomes de cellules squameuses humaines quel que soit leur tissu d'origine.

11. Anticorps selon l'une quelconque des revendications 1-10, ou un de ses fragments, qui réagit avec des lésions prémalignes de l'épithélium pavimenteux.

12. Anticorps selon l'une quelconque des revendications 1-11, ou un de ses fragments, ledit anticorps ou fragment étant marqué ou sous forme solubilisée.

13. Application d'un anticorps monoclonal radiomarqué ou d'un de ses fragments selon l'une quelconque des revendications 1-11 à la préparation d'une composition pour déterminer la localisation d'un carcinome de cellules squameuses chez un malade en produisant une image immunologique dudit anticorps ou fragment d'anticorps monoclonal radiomarqué.

14. Application d'un quantité thérapeutiquement efficace d'un anticorps monoclonal ou d'un de ses fragments selon l'une quelconque des revendications 1-11, ledit anticorps ou fragment d'anticorps monoclonal étant facultativement conjugué avec un agent cytotoxique mais conservant sa spécificité, à la préparation d'une composition pour traiter le carcinome de cellules squameuses chez un malade.

15. Procédé de détection de la présence d'un antigène associé au carcinome de cellules squameuses dans un échantillon, dans lequel on fait incuber un anticorps ou fragment d'anticorps monoclonal selon l'une quelconque des revendications 1-12 avec ledit échantillon pendant une durée suffisante pour permettre à au moins une partie de l'anticorps de se lier audit antigène et de détecter l'anticorps lié.

16. Procédé pour isoler des antigènes qui se lient à un anticorps ou à un de ses fragments selon l'une quelconque des revendications 1-11, dans lequel on immobilise l'anticorps ou son fragment sur un support solide, on met en contact ledit support solide avec un échantillon biologique, on sépare les impuretés non liées d'avec ledit support, et on élue l'antigène sous une forme purifiée à partir de l'anticorps ou fragment immobilisé.

17. Procédé de diagnostic du carcinome de cellules squameuses qui comprend la détection ou la quantification de la réaction de liaison in vitro de l'anticorps ou de son fragment selon l'une quelconque des revendications 1-12 et d'antigènes associés au carcinome de cellules squameuses qui sont liés par l'anticorps ou son fragment.

18. Procédé de détection d'une lésion prémaligne, comme la néoplasie intraépithéliale cervicale ou la kilocytose atypique, de l'épithélium pavimenteux dans un échantillon de tissu, dans lequel on fait réagir l'échantillon avec un anticorps ou fragment selon l'une quelconque des revendications 1-11 et on détermine s'il se produit une liaison ou non.

19. Agent thérapeutique comprenant un anticorps ou fragment selon l'une quelconque des revendications 1-10, ledit anticorps ou fragment étant conjugué avec un agent cytotoxique comme la daunomycine, ledit anticorps ou fragment conservant essentiellement sa spécificité.
